Europäisches Patentamt

European Patent Office

Office européen des brevets

(11) Publication number: **0 271 810 B1**

# EUROPEAN PATENT SPECIFICATION

(45) Date of publication of patent specification: **09.03.94**

(21) Application number: **87118183.0**

(22) Date of filing: **08.12.87**

(51) Int. Cl.5: **G01N 33/86**, G01N 33/564,
G01N 33/577, G01N 33/543,
C07K 15/00, C07K 17/00,
//C07K3/12,C12P21/00,
C12N15/00

(54) Immunological determination of free human protein s and c4bp-protein s complex.

(30) Priority: **15.12.86 JP 296766/86**
**17.12.86 JP 298881/86**

(43) Date of publication of application:
**22.06.88 Bulletin 88/25**

(45) Publication of the grant of the patent:
**09.03.94 Bulletin 94/10**

(84) Designated Contracting States:
**BE CH DE FR GB LI SE**

(56) References cited:
**EP-A- 0 045 103**
**EP-A- 0 128 696**

**CHEMICAL ABSTRACTS, vol. 105, no. 3, 21
July 1986, Columbus, OH (US); R.D. LITWIL-
LER et al., p. 504, no. 22743h&NUM;**

**CHEMICAL ABSTRACTS, vol. 103, no. 1, 08
July 1985, Columbus, OH (US); R.M. BERTINA
et al., p. 242, no. 2587r&NUM;**

(73) Proprietor: **TEIJIN LIMITED**
**11 Minamihonmachi 1-chome**
**Higashi-ku**
**Osaka-shi Osaka 541(JP)**

(72) Inventor: **Koike, Yukiya**
**399-1, Miya**
**Hino-shi Tokyo(JP)**
Inventor: **Wakabayashi, Kenji**
**3-5-18, Tamadaira**
**Hino-shi Tokyo(JP)**
Inventor: **Sumi, Yoshihiko**
**3-18-4, Tamadaira**
**Hino-shi Tokyo(JP)**
Inventor: **Ichikawa, Yataro**
**2-11-7, Kitesashi**
**Tokorozawa-shi Saitama-ken(JP)**

(74) Representative: **Kraus, Walter, Dr. et al**
**Patentanwälte Kraus, Weisert & Partner**
**Thomas-Wimmer-Ring 15**
**D-80539 München (DE)**

CHEMICAL ABSTRACTS, vol. 105, no. 21, 24 November 1986, Columbus, OH (US); R. JENNY et al., p. 355, no. 186874c&NUM;

## Description

This invention relates to the determination of free human protein S or a complex of human protein S and human complement cofactor C4b-binding protein (C4bp) in an assay sample. More specifically, it relates to a method of immunological determination of free human protein S or the aforesaid complex in an assay sample by the "sandwich" technique using a novel specific monoclonal antibody which binds to free human protein S or the aforesaid complex, a novel antibody that can be used in the above method, a reagent system used in the above method.

Protein S, like protein C, is a vitamin K-dependent protein. It was first isolated by Discipio et al. from bovines and humans in 1977 [see Discipio, R. G., Hermodson, M. A., Yates, S. G. and Davie, E. M.: Biochemistry, 16, 696-706 (1977)].

Protein S is a single-chain glycoprotein having a molecular weight of 69,000 (human) which is contained in an amount of about 10 mg/liter in a normal human plasma sample. Its structure is very similar to those of other vitamin K-dependent factors, and it has about 10 gamma-carboxyglutamic acid (Gla) moieties at the $NH_2$ terminus. Protein S exists in two forms in the blood. One is free protein S which acts as a cofactor of activated protein C. The other is a complex of protein S and complement cofactor C4b-binding protein (C4bp), in which protein S is non-covalently bonded to high-molecular C4b binding protein (C4bp), a control factor in a complement system (the complex will be abbreviated sometimes as "C4bp-protein S complex"). The ratio of free protein S to C4bp-protein complex is about 1:1

The importance of the function of protein S in the C4bp-protein S complex is that it has a very strong affinity for a negatively charged surface of a phospholipid [Nelsestuen, G. L., Kisiel, W. and Discipio, R. G.: Biochemistry, 17: 2134-2138 (1978)]. It is believed that when a cell is damaged or activated, the Gla-domain in protein S binds to the phospholipid in the presence of $Ca^{2+}$, and C4bp further binds to protein S to form a complex, whereby the protein S performs its function [Dahlback, KB: Semin. Thromb. Haemostas., 10, 139-148 (1984)].

Recent studies have shown that protein S plays a very important role in the mechanism of controlling the coagulation and fibrinolysis system of protein C, and its physiological significance in relation to thrombosis has aroused interest. It was reported that the congenital deficiency of protein S could be a cause of thrombosis [Comp. P. C., Nixon, R. R., Cooper, N. R. and Esmon, C. T.: J. Clin. Invest., 74: 2082-2088, (1984)].

Chemical Abstracts, Vol. 103 (1), abstr. # 2587 discloses a polyclonal-antibody-based immunoradiometric assay which measures the total amount of protein S, i.e. the sum of the free and the complexed form.

Chemical Abstracts, Vol. 105 (21), abstr. # 186874 discloses, inter alia, a monoclonal antibody (mab) recognizing the protein S/C4bp complex, the use of this mab in a selective adsorbent for homogeneous protein S is also disclosed.

Chemical Abstracts, Vol. 105 (3), abstr. # 22743 discloses, inter alia, a monoclonal antibody recognizing only the free form of protein S.

If it is possible to elucidate the mechanism of action of protein S and to measure the amounts of antigen and activity of protein S in blood, it would have a very important significance in the fields of basic medicine and clinical medicine.

On the other hand, monoclonal antibodies have recently gained widespread use for analysis of the functions and structures of antigen proteins or for immunoassays (EIA, RIA) because of the advantage that they are specific only for a single epitope and monoclonal antibodies having the same specificity can be produced stably. Particularly, for the functional and molecular analysis of antigen proteins, it will be a suitable means for discovering an antibody which recognizes a site that is involved in the function of the antigen proteins or their special structural sites.

Conventional methods for determining protein S include, for example, the Laurell method using an antiserum to protein C, and RIA (radioimmunoassay) and EIA (enzyme immunoassay) involving the use of polyclonal antibodies. These methods, however, permit measurement of only the total amount of free protein S and its complex with C4bp. The former method has the disadvantage that animal antisera having a fixed activity are extremely difficult to obtain stably in large amounts, and the activity of the sera must be corrected by using a standard substance. It also has the defect that a long period of time is required for immunodiffusion. According to the latter methods, antibodies must be purified from antisera, and stable antibodies are difficult to obtain steadily.

The present inventors noted that protein S forms a complex with complement cofactor C4b-binding protein, C4bp, and thought that if a monoclonal antibody which binds selectively only to free protein S and a monoclonal antibody which binds selectively only to the C4bp-protein S complex can be obtained, it

3

would be possible to perform selective immunological determination of free protein S and C4bp-protein S complex simply and accurately without the defects of the prior art. Extensive investigations based on this thought have now led to the successful creation of specific monoclonal antibodies.

According to one aspect of this invention, there is provided a method of immunologically determining free human protein S in an assay sample, which comprises contacting a primary antibody fixed to an insoluble solid carrier and a labelled secondary antibody with the assay sample, the primary and secondary antibodies having the property of binding to different epitopes of free human protein S, and one of the primary and secondary antibodies being a monoclonal antibody having the property of not binding to a complex of the human protein S and human complement cofactor C4b-binding protein (C4bp) but specifically binding to the free human protein S.

According to another aspect of this invention, there is provided a method of immunologically determining a complex of human protein S and human complement cofactor C4b-binding protein (C4bp) in an assay sample, which comprises contacting a primary antibody fixed to an insoluble solid carrier and a labelled secondary antibody with the assay sample, one of the primary and secondary antibodies being a monoclonal antibody having the property of not binding to free human protein S and human complement cofactor C4b-binding protein (C4bp) but binding specifically to said complex, and the other being an antibody having the property of binding to the human complement cofactor C4b-binding protein (C4bp).

The method of immunologically determining a specific antigen in an assay sample using a primary antibody fixed to an insoluble solid carrier and a labelled secondary antibody is called the "sandwich method" and is described for example, in Wide "Radioimmunoassay Methods", 199-206 (1970).

The methods provided by this invention are characterized by the fact that based on the principle of the sandwich method, one of free human protein S and C4bp-protein S complex in an assay sample is selectively determined by using the following monoclonal antibodies created newly by the present inventors.

(a) A monoclonal antibody having the property of not binding to C4bp-protein S complex but specifically binding to free human protein S (to be referred to as "monoclonal antibody A" for the sake of convenience in the present specification).

(b) A monoclonal antibody having the property of not binding to free human protein S and C4bp but specifically binding to C4bp-protein S complex (to be referred to as "monoclonal antibody B" for the sake of convenience in the present specification).

These methods of the invention permit measurement of free human protein S or C4bp-protein S complex in an assay sample (for example, plasma) with constant good accuracy without variations in the quality of reagents. According to the methods of this invention, free human protein S and C4bp-protein S complex can be measured directly, and accurate measurement within short periods of time can be carried out without any influence of foreign materials in the sample.

The determination methods of this invention thus permit diagnosis of the conditions of thrombosis having cancer as a basic disease, nephrosis, etc., and accurate determination of the fibrinolytic state of toxemia of pregnancy and the fibrinolytic state of patients having undergone surgical operation, which have heretofore been substantially impossible. This offers great advantages to medicine.

The invention will be described in greater detail.

(1) Determination of free human protein S in an assay sample by using the monoclonal antibody A:-

In the method of immunologically determining free human protein S in an assay sample according to this invention, one (primary antibody) of the two antibodies is fixed to an insoluble solid carrier, and the other (secondary antibody) is used in the labelled state. The monoclonal antibody may be used as the primary antibody fixed to the insoluble solid carrier, or as the labelled secondary antibody. In either case, there is substantially no effect on the results of determination of human protein S.

The antibody used in combination with the monoclonal antibody A may be any monoclonal or polyclonal antibody which recognizes an epitope of human protein S different from that recognized by the monoclonal antibody A and binds to it. Generally, however, it is convenient to use a monoclonal antibody which recognizes and binds to an epitope of free human protein S different from that recognized by the monoclonal antibody A, and which is produced from a hybridoma obtained as a by-product during screening of a hybridoma producing the monoclonal antibody A.

The primary antibody may be fixed to the insoluble solid carrier by methods known per se. For example, a solution of the primary antibody and the insoluble solid carrier are contacted and left to stand, whereby the antibody is physically adsorbed on the carrier. It is also possible to combine the functional groups of the antibody, such as a carboxyl, amino or hydroxyl group, chemically with the insoluble solid carrier. Preferably, the surface of the carrier to which the primary antibody has been fixed is coated with a suitable substance such as bovine serum albumin in order to avoid non-specific combination with the secondary antibody or the assay sample.

Examples of the insoluble solid carrier used to fix the primary antibody include polymeric materials such as polystyrene, polyethylene, polypropylene, polyesters, polyacrylonitrile, fluorine-containing resins, nitrocellulose, crosslinked dextran, polysaccharides and agarose, inorganic materials such as glass and metal, and combinations of these. The solid carrier may be in various shapes, for example in the shape of a tray, sphere, fiber, particle, bead, disc, rod, receptacle, cell or test tube. Specific examples of the insoluble solid carrier are plastic receptacles, plastic beads, glass beads and metal particles.

The secondary antibody is labelled with radioisotopes, enzymes or luminescent substances. Examples of the radioisotopes are $^{125}I$, $^{131}I$, $^{14}C$ and $^{3}H$. Examples of the enzymes are alkaline phosphatase, peroxidase, and beta-D-galactosidase. Examples of the luminescent substances are fluorescein isothiocyanate and tetramethyl rhodamine isothiocyanate. These are merely illustrative, and other labelling substances used in immunological assays may also be used. Combination of the labelling substances with the secondary antibody may be effected by methods known per se, for example by the methods described in G. S. David: Biochem. Biophys. Res. Commun., 48, 464-471 (1972), M. Imagawa et al., Anal. Lett., 16, 1509-1523 (1983) and M. Nishioka et al., Cancer Res., 32, 162-166 (1972).

The fixed primary antibody and the labelled secondary antibody are then brought into contact with an assay sample for determination of human protein S by a two-step method comprising contacting the sample first with the fixed primary antibody and then with the labelled secondary antibody, or by a one-step method comprising contacting the sample and the secondary antibody simultaneously with the primary antibody. The one-step method is advantageous over the two-step method because it permits a simpler and more rapid determination of human protein S.

In the two-step method, the fixed primary antibody and the sample are contacted and reacted at a given temperature for a given period of time. During this time, the fixed primary antibody combines with the human protein S in the sample. After washing with a suitable washing liquor, the reaction product is contacted and reacted with a solution (e.g., an aqueous solution) of the labelled secondary antibody at a given temperature for a given period of time. The reaction product is washed with a suitable washing liquor, and the amount of the labelling substance present on the insoluble solid carrier is measured. The amount of the human protein S in the sample can be determined by comparing the amount of the labelling substance with a calibration curve drawn by using an assay sample containing human protein S in a known concentration.

In the one-step method, the fixed primary antibody is contacted and reacted with the assay sample and the labelled secondary antibody simultaneously, preferably with a mixture of the sample and the labelled secondary antibody, at a given temperature for a given period of time. The product is then washed with a suitable washing liquor, and the amount of the labelling substance present on the insoluble solid carrier is measured as described above. As a result, the amount of human protein S in the sample can be determined.

According to the methods described above, the amount of human protein S in the assay sample can be measured easily with good reproducibility and a high accuracy. Human plasma, human serum and a supernatant from a cell culture are examples of the sample which can be assayed by the above methods.

For the practice of the above method, the present invention provides a reagent system comprising the primary antibody fixed to the insoluble solid carrier and the labelled secondary antibody. A kit may be formed from this reagent system and various auxiliary agents in order to use the reagent system efficiently and easily. Examples of the auxiliary agents include dissolving agents for dissolving the solid secondary antibody, washing agents for washing the insoluble carrier, substrates for measuring the enzyme activity of enzymes which may be used as labelling substances for the secondary antibody, and reaction stoppers therefor, which are normally used in reagent kits for immunological assay.

(2) Determination of a human C4bp-protein S complex in an assay sample by using the monoclonal antibody B:-

The method of immunologically determining human C4bp-protein S complex in an assay sample according to this invention and the reagent system used therefor may be the same as the method and reagent system described in (1) above except that in the reagent system, the monoclonal antibody B is used instead of the monoclonal antibody A, and a monoclonal or polyclonal antibody capable of recognizing and binding to C4bp is used instead of the monoclonal or polyclonal antibody which recognizes and binds to an epitope of human protein S different from that recognized by the monoclonal antibody A. Repetition of the description of the method will therefore be omitted.

(3) Monoclonal antibodies A and B and preparation thereof

The monoclonal antibody A or B can be obtained by establishing a hybridoma cell line capable of producing such antibody, and then cultivating the hybridoma.

The hybridoma capable of producing the monoclonal antibody A or B can be produced by a technique known as the Köhler and Milstein method [Köhler and Milstein, Nature, 256, 495-497 (1975)]. Specifically, a mammal such as a mouse is immunized with free human protein S, and antibody-producing cells, for example, spleen cells, of this animal are fused with myeloma cells. The fused cells are screened by cloning for fused cells capable of producing the monoclonal antibody of this invention. For example, the fused cells produced are systematically screened for an antibody which reacts with free human protein S or human C4bp-protein S complex fixed to microtiter plates.

The monoclonal antibody A or B can be obtained from the product yielded by this hybridoma. The resulting monoclonal antibody acts monospecifically on the epitope of free human protein S or C4bp-protein S complex.

The monoclonal antibody A or B and a process for producing it will now be described in more detail.

(A) Isolation and purification of an antigen

Free human protein S used as an antigen is isolated in pure form from a human plasma sample by the method of BaJaj et al. [BaJaj S. P, Rapaport S. I., Maki S. L., Brown S. F.: Prep. Biochem., 13, 191, (1983)].

(B) Immunization of mammals with free human protein S

There is no particular restriction on the animals to be immmunized, and various mammals such as mice, rats, guinea pigs, rabbits, sheep, goats, dogs and cats may be used. For the ease of handling, male Balb/c mice are generally used. Mice of other strains may also be used. The immunization should be planned, and the concentration of free human protein S to be used in immunization should be selected, so that sufficient amounts of antigenically stimulated lymphocytes can be formed. For example, a mouse is intraperitoneally immunized several times with a small amount of free human protein S at certain intervals, and the antigen is further administered intravenously several times to increase the titer of the antibody. Several days after the final immunization, antibody-producing cells, for example, lymphocytes, preferably spleen cells, are taken out from the immunized animals. The following description is given with regard to the use of spleen cells as the antibody-producing cells, but it should be understood that other antibody-producing cells isolated from immunized animals can equally be used for cell fusion.

(C) Cell fusion

The spleen is aseptically taken out from the immunized animal, and a spleen cell suspension is prepared from it. The spleen cells are then fused with myeloma cells taken from a suitable cell line in a fusion medium in the presence of a suitable fusion promoter. The myeloma cells used for fusion may be obtained from any mammals, but generally, those originated from the same kind of animal as the immunized animal are preferred. The preferred mixing ratio of the spleen cells to the myeloma cells is generally in the range of from about 20:1 to about 2:1, preferably from 10:1 to 2:1. Usually, the use of 0.5 to 1.5 ml of the fusion medium per about $10^8$ spleen cells is suitable. Suitable fusion media are, for example, physiological saline, buffered saline, a serum-free medium each of which contains the fusion promoter in a concentration of 30 to 70%.

Many myeloma cells suitable for cell fusion are known. In Examples to be given hereinafter, P3-X63-Ag8-U1 cells (to be abbreviated as P3-U1) [see D. E. Yelton et al.: Current Topics in Microbiology and Immunology, 81, 1 (1978)]. They are an 8-azaguanine resistant cell line. They lack hypoxanthine-guanine phosphoribosyl transferase, and therefore do not survive in HAT medium (containing hypoxanthine, aminopterin and thymidine). Furthermore, since this cell line is of a non-secreting type which does not secrete an antibody itself, it is suitable for the production of the hybridoma contemplated by the present invention. Other myeloma cells may also be used. Examples include P3-NS1-1-Ag4-1, NS1-Ag4/1, P3-X63-Ag8, (MPCH-45, 6. TG1.7), SP2/0-Ag14, FO, X-63-Ag8-6.5.3, 210.RCY3.Ag1.2.3, S194/5XXO.BU.1, SKO-007, and GM15006TG-A12.

6

Polyethylene glycol having an average molecular weight of 1,000 to 4,000, for example, may be advantageously used as the fusion promoter. There can also be used other fusion promoters known in the art, such as Sendai virus. In the following Examples, polyethylene glycol having an average molecular weight of 1,540 was used.

(D) Detection of the fused cells

A mixture of the fused cells, non-fused spleen cells and non-fused myeloma cells is diluted in a separate receptacle (such as a microtiter plate) with a selective medium in which the non-fused myeloma cells cannot survive, and cultivated for a sufficient period of time to allow the non-fused cells to die (about 1 week). The culture medium may be one which is resistant to a drug such as 8-azaguanine and in which the non-fused myeloma cells cannot survive, for example the aforesaid HAT medium. In the selective medium, the non-fused myeloma cells die away. Since the non-fused spleen cells are non-tumoral, they die after a certain period of time (about 1 week). On the other hand, the fused cells can survive in the selective medium because they have both the tumor-bearing nature of the parent myeloma cells and the nature of the parent spleen cells.

(E) Determination of an antibody to free human protein S in each receptacle:-

After the hybridoma cells are detected as stated above, the supernatant of the culture fluid is collected, and screened for an antibody to free human protein S or C4bp-protein S complex by enzyme linked immunosorbent assay (see, for example, A. H. W. M. Schuurs and B. K. van Weemen: Clin. Chim. Acta, 81, 1-40 (1977)].

(F) Cloning of the hybridoma capable of producing the antibody A or B

The hybridoma capable of producing the desired antibody can be cloned by a suitable method such as a limiting dilution method in two different ways. In one way, the hybridoma is cultivated in a suitable medium for a given period of time, and the monoclonal antibody produced by the hybridoma can be obtained from the supernatant of the culture fluid. In the other, the hybridoma can be intraperitoneally injected into a syngenic mouse. After a certain period of time, the monoclonal antibody A or B produced by the hybridoma can be obtained from the blood and ascites of the host animal.

(G) Properties of the monoclonal antibodies A and B

The monoclonal antibody A produced as above has an isoelectric point in the range between a pH of 6.2 and 6.8 and binds selectively only to free human protein S. The class of its H-chains is $\gamma_1$, and the class of its L-chains is $x$. Its dissociation constant (KD), measured by the method of Frankel and Gerhard [Frankel, M. E., Gerhard, W.: Mol. Immunol. 16, 107 (1979)], is is $0.5\text{-}1.5 \times 10^{-9}$ M with respect to free protein S, but cannot be measured with respect to C4bp-protein S complex because it does not substantially bind to the complex.

The monoclonal antibody B has an isoelectric point in the range between a pH of 5.6 and 6.2, and binds selectively only to C4bp-protein S complex (namely does not substantially bind to free protein S nor to C4bp). The class of its H-chains is $\gamma_{2b}$, and the class of its L-chains is $x$. The dissociation constant (KD) of the monoclonal antibody B, measured by the method of Frankel and Gerhard, is $4.0\text{-}6.0 \times 10^{-8}$ M with respect to free protein S, and $1.0\text{-}2.0 \times 10^{-9}$ M with respect to C4bp-protein S complex. Its dissociation constant with resepct to C4bp cannot be measured because it does not substantially bind to C4bp (the dissociation constant with respect to free protein S cannot be measured by the EIA method because it is outside the detection limit).

Litwiller et al. [1] and Malm et al. [2] separately reported monoclonal antibodies to human protein S specific for a $Ca^{2+}$-stabilized epitope [[1] R. D. Litwiller, R. J. Henny, J. A. Katzmann, R. S. Miller, and K. C. Mann: Blood, vol. 67, No. 6, pp. 1583-1590 (1986); [2] J. Malm, U. Persson and B. Dahlback: European Journal of Biochemistry, vol. 165, pp. 39-45 (1987)]. The epitopes for these monoclonal antibodies are located in the Gla region of protein S or in a closely positioned thrombin-sensitive region. These monoclonal antibodies recognize both free protein S and the C4bp-protein S complex. The monoclonal antibodies A and B are used in the method of the present invention. The monoclonal antibody A recognizes only free protein S, and does not bind to the C4bp-protein S complex, whereas the monoclonal antibody B recognizes the C4bp-protein S complex specifically. This fact demonstrates that the monoclonal antibodies A and B are different and distinguished from the monoclonal antibodies of Litwiller et al. and Malm et al.

The monoclonal antibody A can also be applied to the separation or recovery of free human protein S from a liquid containing the free human protein S because it has the function of specifically binding to the epitope of the free human protein S.

Brief Description of the Accompanying Drawings

Fig. 1 shows the strengths of binding of the monoclonal antibodies used in the method of the present invention to human protein S.

Fig. 2 shows the strengths of binding of the monoclonal antibodies used in the method of the present invention to the C4bp-protein S complex.

Fig. 3 shows the isoelectric points of the monoclonal antibodies used in the method of the invention.

Fig. 4 is a calibration curve showing the relation between the concentration of human protein S and changes in absorbance.

Fig. 5 is a calibration curve showing the relation between the amount of the human C4bp-protein S complex and changes in absorbance.

Fig. 6 shows the actual measured amounts of human protein S and human C4bp-protein S complex in plasma samples taken from human patients.

The following Examples illustrate the present invention in great detail. In these Examples, protein S is sometimes abbreviated as "PS".

EXAMPLE 1

Two female Balb/C mice (four weeks old) were immunized four times with purified human PS at 14-day intervals. In the first immunization, 50 micrograms of human PS dissolved in PBS was mixed with an equal quantity of complete Freund's adjuvant, and the resulting emulsion was intraperitoneally administered (0.5 mg/head). In the second and third immunizations, 50 micrograms of human PS was mixed similarly with Freund's incomplete adjuvant, and intraperitoneally administered. In the final immunization, 30 micrograms of human PS in PBS was directly administered through the tail vein. Three days after the final immunization, the spleen cells of the immunized mice were used in cell fusion.

The spleen cells of the immunized mice and myeloma cells (P3U1) of mice of the same strain were mixed at a ratio of from about 5:1 to about 7:1 and fused in accordance with the method of Köhler and Milstein in the presence of 50% ethylene glycol 1540 (a product of Wako Pure Chemicals Co., Ltd.). The fused cells were suspended in 10% FCS-RPM1-1640 medium so that the cell concentration was 1 x 10$^6$ cells/ml. The suspension was poured onto a 95-well microplate (made by Coster Company) at a rate of 100 microliters per well.

The fused cells were cultivated in an CO$_2$ incubator (5% CO$_2$, 37°C). The medium was replaced by a medium containing hypoxanthine, aminopterin and thymidine (HAT medium), and the cells were grown in the HAT medium. Hybridomas composed of the spleen cells and the myeloma cells were screened.

The antibodies in the supernatant of the culture fluid of the hybridoma were detected by the ELISA method using microtiter plates coated with antigen human PS. Alkaline phosphatase-conjugated rabbit anti-mouse IgG antibody was used as a second antibody, and the bindability of the antibodies to the antigen PS was examined. Among 494 wells in total in which the fused cells had been seeded, formation of colonies was observed in 437 wells. Of these, 94 wells were positive in antibody production showing the ability to bind to the antigen PS.

On four wells out of the positive wells, cloning was carried out twice by the limiting dilution method, and six clones were obtained. The clones obtained were suspended in 90% FCS-10% DMSO and stored in liquid nitrogen.

Monoclonal antibodies produced by these clones were grown in the abdominal cavities of Balb/C mice, and purified from the ascites of the mice using a protein A-Sepharose 4B column.

### Table 1 (cell fusion)

| | Cell fusion 1 | Cell fusion 2 | Total |
|---|---|---|---|
| Spleen cells (per ml) | $1.15 \times 10^7$ | $6.70 \times 10^6$ | |
| Myeloma cells (per ml) | $1.72 \times 10^6$ | $1.33 \times 10^6$ | |
| Ratio of spleen cells/myeloma cells | 6.0 | 5.0 | |
| Number of cells per well | $0.57 \times 10^5$ | $0.67 \times 10^5$ | |
| Number of wells | 302 | 192 | 494 |
| Wells positive in colony formation | 298 (98.6%) | 189 (98.4%) | 487 (98.6%) |
| Wells positive in antibody production | 66 (21.8%) | 28 (14.8%) | 94 (19.0%) |

EXAMPLE 2

Properties of the purified monoclonal antibodies:-

The clones purified from the mouse ascites were examined for IgG classes and bindability to human protein S (PS).

The particular classes of the mouse monoclonal antibodies were determined by the Ouchterlony method using anti-mouse anti-sera specific for the individual classes.

The results are shown in Table 2.

The bindability of the antibodies to human protein S was evaluated by reacting human protein S fixed to microtiter plates with the monoclonal antibodies diluted to suitable concentrations and detecting the reaction products by using alkaline phosphatase-conjugated goat anti-mouse IgG.

The strengths of binding of the six monoclonal antibodies to human protein S are: $2B9F12 \cong 2B9C10 > 3C3G8 > 3C4G4 > 2B9G2 >> 2E12C7$.

9

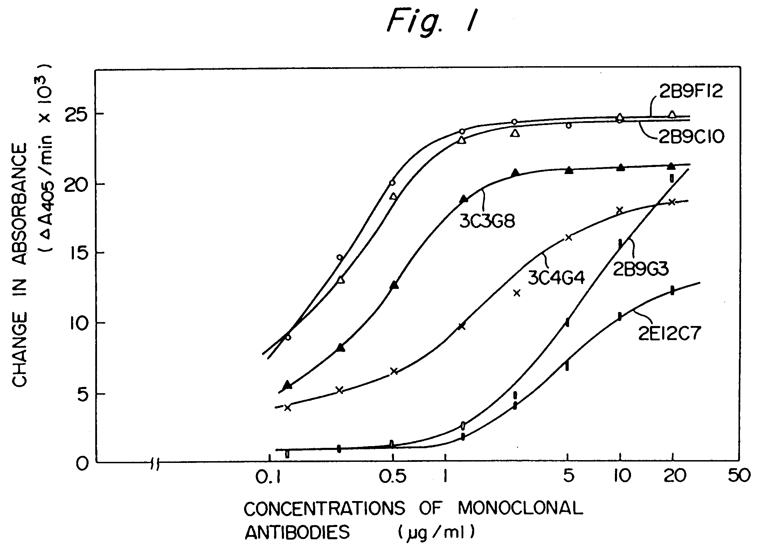

The results are shown in Fig. 1.

## Table 2

| Monoclonal antibody | Class |
|---|---|
| 2B9F12 | $IgG_1$ |
| 2B9C10 | $IgG_1$ |
| 3C3G8 | $IgG_3$ |
| 3C4G4 | $IgG_1$ |
| 2B9G3 | $IgG_1$ |
| 2E12C7 | $IgG_{2b}$ |

EXAMPLE 3

Reactivity with human C4b-binding protein and protein S complex (C4bp-PS complex):-

Three purified monoclonal antibodies (2B9F12, 2B9C10, 2E12C7) and goat anti-PS antibody were coated on microtiter plates in a concentration of 10 micrograms/ml, and blocked with 1% BSA. Then, the C4bp-protein S complex diluted to a suitable concentration was added and reacted with the monoclonal antibodies. Then, alkaline phosphatase-conjugated anti-C4bp antibody was added and the bindabilities of the three monoclonal antibodies to the C4bp-protein S complex were detected.

Monoclonal antibody 2E12C7 had very weak bindability to free protein S, but showed a high degree of specific bindability to the C4bp-protein S complex. On the other hand, 2B9F12 did not show bindability to the C4bp-protein S comlex.

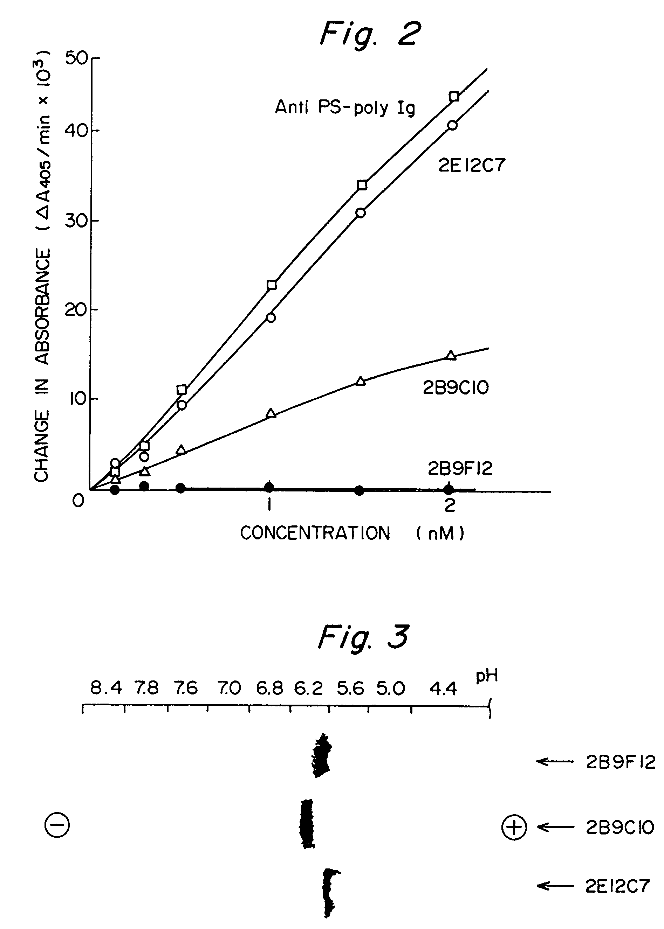

The results are shown in Fig. 2

In this way, by using various antibodies in immunological assaying means (EIA, RIA), free human protein S and C4bp-protein S complex in solution (for example, human plasma) can be assayed.

EXAMPLE 4

Measurement of dissociation constants (KD):-

A purified monoclonal antibody was labelled with $^{125}I$ (iodine-125) by using Immunobeads (a product of Bio-Rad Co.).

Purified protein S and C4bp-protein S complex in a concentration of 1 microgram/ml were added to a 96-well microtiter plate (Titertek made by Flow Lab. Co.) at a rate of 50 microliters/well, and adsorbed at 4°C overnight. 10 mM phosphate buffer (pH 7.2) containing 1% BSA and 0.125 M NaCl was added at a rate of 100 microliters/well. The plates were then left to stand at room temperature for 2 hours, and then washed twice (100 microliters/well) with 10 mM phosphate buffer (pH 7.2) containing 0.05% Tween 20 (washing solution). The $^{125}I$-labelled monoclonal antibody diluted to various concentrations (0.01 to 5 micrograms/ml) was added to the wells of the plate and incubated at 37°C for 2 hours. The plate was then washed three times with the above washing solution (100 microliters/well). The wells were cut out from the plate and put in plastic test tubes. The $^{125}I$-radioactivity was measured by a gamma counter (radioactivity bound to the solidified antigen: B in cpm). At the same time, the radioactivity of the diluted solution of $^{125}I$-labelled monoclonal antibody solution added to the wells was measured (the total added radioactivity: T in cpm). The concentration of the added monoclonal antibody was taken on the axis of abscissa, and the ratio between T-B (the radioactivity not bound to the solidified antigen: F in cpm) and B, on the axis of ordinates. From the Scatchard plot, the dissociation constant (KD) was calculated. The results are summarized in Table 3.

## Table 3

| Antibody | PS | PS-C4bp |
|----------|-----|---------|
| | KD (mole/liter) | (KD (moles/liter) |
| 2B9F12 | $1.03 \times 10^{-9}$ | Not detected |
| 2B9C10 | $4.89 \times 10^{-9}$ | $7.29 \times 10^{-8}$ |
| 2E12C7 | $5.71 \times 10^{-8}$ | $1.84 \times 10^{-9}$ |

EXAMPLE 5

Measurement of the isoelectric points of purified monoclonal antibodies:-

Each of purified monoclonal antibodies (2B9F12, 2B9C10 and 2E12C7) in an amount of 20 micrograms was subjected to agarose isoelectric focusing at 4°C under a pH gradient of 3.5 to 9.0. After isoelectric focusing for 2 hours, the antibody protein in the agarose gel was immobilized and stained with CBB. The photograph of this electrophoresis was taken and shown in Fig. 3. From the positions of the electrophoretic bands of the monoclonal antibodies, the isoelectric points of the antibodies were measured using a standard isoelectric point marker electrophoresed simultaneously. They were a pH of 6.2 to 6.8 for 2B9F12, a pH of 6.2 to 6.8 for 2B9C10, and a pH of 5.6 to 6.2 for 2E12C7.

EXAMPLE 6

(1) Fixing of an antibody to an insoluble carrier

A dry gel (0.5 g) of cyanogen bromide-activated Sepharose 4B (made by Pharmacia Fine Chemicals, Inc.) was swollen and washed with 100 ml of 1 mM HCl and then further washed with a coupling buffer (0.1 M NaHCO₃ containing 0.5 M NaCl, pH 8.3) on a G3 glass filter. The coupling buffer was removed by suction, and immediately then, the gel was suspended in 2 ml of a coupling buffer solution (3 mg/ml) for a monoclonal antibody (2B9F12). The suspension was gently shaken overnight at 4°C. The gel was then transferred to 1 M ethanolamine-HCl (pH 8.0, 2 ml) and shaken at room temperature for 2 hours to block the remaining active groups. After the blocking, the antibody-bound Sepharose gel was washed on a glass filter with 0.1 M acetate buffer (pH 4.0) containing 0.5 M NaCl and 0.1 M borate buffer (pH 8.0) containing 0.5 M NaCl alternately. When the absorbance of the filtrate at 280 nm decreased below 0.01, it was equilibrated with 0.05 M Tris/HCl (pH 7.4) containing 1 mM benzamidine, and filled in a column. Affinity chromatography was performed on the prepared anti-human PS monoclonal antibody (2B9F12)-bound Sepharose 4B column.

(2) Adsorption of protein S on antibody-bound Sepharose 4B and elution thereof

To 100 ml of plasma was added to 8 ml of 1M BaCl₂ solution, and the mixture was stirred at 4°C for 1 hour. The precipitate was collected by centrifugal separation, and washed with 0.1 M NaCl containing 5 mM BaCl₂ and 5 mM benzamidine. The precipitate was dissolved in 15 ml of 0.2 M EDTA (pH 7.4) containing 5 mM benzamidine to obtain a barium adsorbed fraction. The barium-adsorbed reaction was dialyzed against 0.05 M Tris/HCl (pH 7.4) containing 1 mM benzamidine and applied to the antibody 2B9F12-bound column equilibrated with 0.05M Tris/HCl (pH 7.4) containing 1 mM benzamidine. The column was washed with 0.05 M Tris/HCl (pH 7.4) containing 1 mM benzamidine and 1 M NaCl and eluted with 3 M NaSCN (pH 7.0) solution. A single peak containing PS was obtained. The recovery ratio was about 75.4%.

EXAMPLE 7

Measurement of human protein S:-

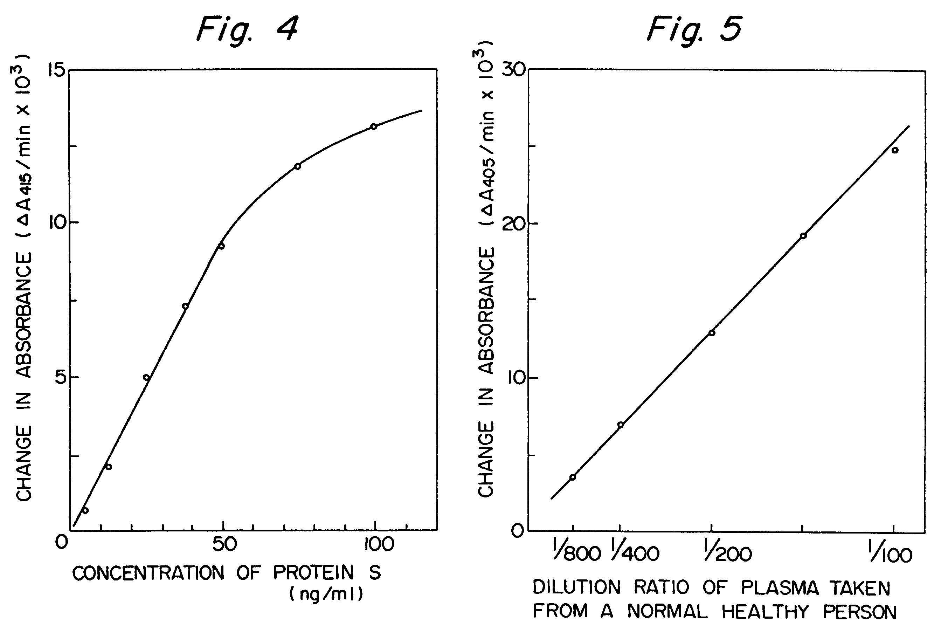

The monoclonal antibody obtained in Example 1 (2B9F12) to human protein S was coated on a microtiter plate in a concentration of 15 micrograms/ml. The plate was blocked with phosphate buffer (PBS) containing 1% bovine serum albumin (BSA), and then washed three times with a washing solution (0.5% BSA-PBS containing 0.05% of Tween). Then, purified protein S and a plasma sample taken from a human patient were diluted to suitable concentrations with PBS and reacted at 37°C for 2 hours with the monoclonal antibody adsorbed on the solid phase of the plate. The plate was washed three times with the washing solution, and then a solution of a peroxidase-labelled monoclonal antibody (2B9C10) to human protein S was added. The plate was incubated at 37°C for 2 hours, and washed with the washing solution three times. Then, a substrate (ABTS) solution was added, and changes in absorbance per minute ($\Delta$A415 nm/min.) was measured by an ELISA ANALYZER (ETY-96 made by Toyo Sokki Co., Ltd.). The concentrations of purified protein S and the changes in absorbnce were plotted to draw a calibration curve. The calibration curve is shown in Fig. 4. The concentration of protein S and the absorbance was in a straight-line relationship up to about 50 ng/ml. If this calibration curve is used, the amount of human protein S can be accurately calculated with high sensitivity.

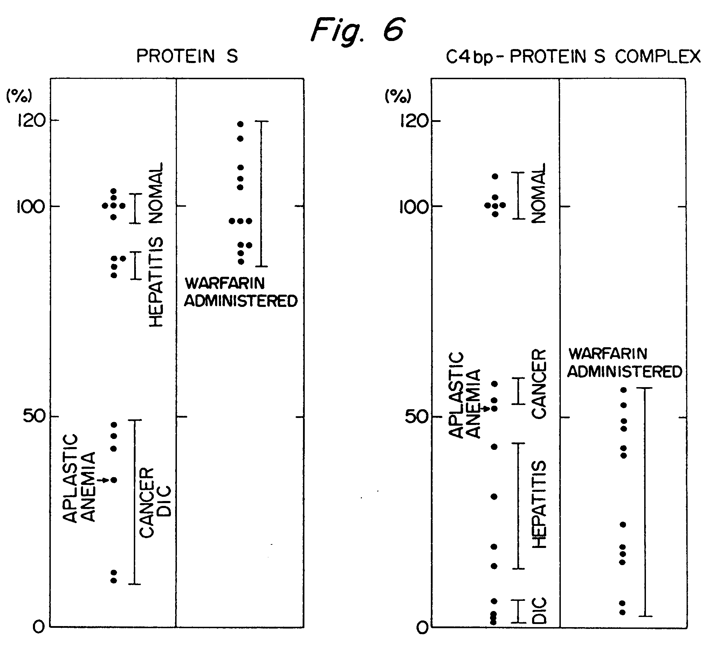

The amount of protein S in plasma samples of human patients was calculated by using this calibration curve, and the results are shown in Fig. 6. In Fig. 6, the amount of protein S in a plasma sample taken from a normal healthy subject is indicated as 100%.

EXAMPLE 8

Measurement of the human C4bp-protein S complex:-

The monoclonal antibody (2E12C7) to human protein S which bound specifically to the human C4bp-protein S complex was coated in a concentration of 20 micrograms/ml on a microtiter plate. The plate was blocked with phosphate buffer (PBS) containing 1% bovine serum albumin (BSA) and then washed three times with a washing solution (0.5% BSA-PBS containing 0.05% of Tween 20).

A plasma sample taken from a normal healthy person and plasma samples taken from patients were diluted to suitable concentrations with PBS, and each reacted at 37°C for 2 hours with the monoclonal antibody adsorbed on the solid phase of the plate. The plate was washed three times with the washing solution, and reacted with a solution of an alkaline phosphatase-labelled polyclonal antibody to human C4b-binding protein (C4bp) at 37°C for 2 hours. The plate was then washed three times with the washing solution, and a substrate solution (1 mg/ml) was added. Changes in absorbance per minute ($\Delta$A405 nm/min.) were measured by an ELISA ANALYZER (ETY-96, made by Toyo Sokki Co., Ltd.). The plasma of a healthy person was diluted, and the amount of the human C4bp-protein S complex in the plasma and the changes in absorbance were plotted to draw a calibration curve. The calibration curve is shown in Fig. 5. The concentration of the human C4bp-protein S complex in the plasma of the healthy person and the changes in absorbance were in a straight-line relationship. The use of this calibration curve permitted accurate calculation of the amount of the human C4bp-protein S with high sensitivity.

The amount of the human C4bp-protein S complex in the plasma samples of human patients was calculated by using this calibration curve, and the results are shown in Fig. 6. In Fig. 6, the amount of the human C4bp-protein S complex in the plasma of the healthy person was indicated as 100%.

It is seen that various diseases can be diagnosed by determining the amounts of free protein S and C4bp-protein S complex in human plasma using monoclonal antibodies to human protein S.

**Claims**

1. A method of immunologically determining free human protein S in an assay sample, which comprises contacting a primary antibody fixed to an insoluble solid carrier and a labelled secondary antibody with the assay sample, the primary and secondary antibodies having the property of binding to different epitopes of free human protein S, and one of the primary and secondary antibodies being a monoclonal antibody having the property of not binding to a complex of the human protein S and human complement cofactor C4b-binding protein (C4bp) but specifically binding to the free human protein S.

2. The method of claim 1 wherein the labelled secondary antibody is labelled with an enzyme, a luminescent substance or a radioisotope.

3. The method of claim 1 wherein the labelled secondary antibody and the assay sample are contacted simultaneously with the primary antibody fixed to an insoluble solid carrier.

4. A method of immunologically determining a complex of human protein S and human complement cofactor C4b-binding protein (C4bp) in an assay sample, which comprises contacting a primary antibody fixed to an insoluble solid carrier and a labelled secondary antibody with the assay sample, one of the primary and secondary antibodies being a monoclonal antibody having the property of not binding to free human protein S and human complement cofactor C4b-binding protein (C4bp) but binding specifically to said complex, and the other being an antibody having the property of binding to the human complement cofactor C4b-binding protein (4Cbp).

5. The method of claim 4 wherein the labelled secondary antibody is labelled with an enzyme, a luminescent substance or a radioisotope.

6. The method of claim 4 wherein the labelled secondary antibody and the assay sample are contacted simultaneously with the primary antibody fixed to an insoluble solid carrier.

7. A reagent system for immunological determination of free human protein S in an assay sample comprising a primary antibody fixed to an insoluble solid carrier and a labelled secondary antibody, wherein the primary and secondary antibodies have the property of binding to different epitopes of free human protein S, and one of the primary and secondary antibodies is a monoclonal antibody having the property of not binding to a complex of the human protein S and human complement cofactor C4b-binding protein (C4bp) but specifically binding to the free human protein S.

8. A reagent system for immunological determination of a complex of human protein S and a human complement cofactor C4b-binding protein (C4bp) in an assay sample comprising a primary antibody fixed to an insoluble solid carrier and a labelled secondary antibody, wherein one of the primary and secondary antibodies is a monoclonal antibody having the property of not binding to free human protein S and human complement cofactor C4b-binding protein (C4bp) but binding specifically to said complex, and the other is an antibody having the property of binding to the human complement cofactor C4b-binding protein (C4bp).

9. The reagent system of claim 7 or 8 wherein the insoluble solid carrier is a plastic receptacle, plastic beads, glass beads or metal particles.

10. The reagent system of claim 7 or 8 wherein the labelled secondary antibody is the monoclonal antibody labelled with an enzyme, a luminescent substance or a radioisotope.

**Patentansprüche**

1. Verfahren zur immunologischen Bestimmung von freiem, menschlichen Protein S in einer Testprobe, **dadurch gekennzeichnet,** daß man einen ersten Antikörper, der an einen unlöslichen, festen Träger fixiert ist, und einen markierten zweiten Antikörper mit der Testprobe in Kontakt bringt, wobei der erste und der zweite Antikörper die Eigenschaft besitzen, an verschiedene Epitope von freiem menschlichen Protein S zu binden und entweder der erste oder der zweite Antikörper ein monoclonaler Antikörper mit der Eigenschaft, nicht an einen Komplex aus dem menschlichen Protein S und dem den menschlichen Komplement-Cofaktor C4b-bindenden Protein (C4bp) zu binden, aber spezifisch an freies menschliches Protein S zu binden, ist.

2. Verfahren nach Anspruch 1, **dadurch gekennzeichnet,** daß der markierte zweite Antikörper mit einem Enzym, einer luminszierenden Substanz oder einem Radioisotop markiert ist.

3. Verfahren nach Anspruch 1, **dadurch gekennzeichnet,** daß der markierte zweite Antikörper und die Testprobe gleichzeitig mit dem ersten Antikörper, der an einen unlöslichen, festen Träger fixiert ist, in Kontakt gebracht werden.

4. Verfahren zur immunologischen Bestimmung eines Komplexes aus menschlichem Protein S und dem den menschlichen Komplement-Cofaktor C4b-bindenden Protein (C4bp) in einer Testprobe, **dadurch gekennzeichnet,** daß man einen ersten Antikörper, der an einen unlöslichen, festen Träger fixiert ist und einen markierten zweiten Antikörper mit der Testprobe in Kontakt bringt, wobei entweder der erste oder der zweite Antikörper ein monoclonaler Antikörper mit der Eigenschaft, nicht an freies menschliches Protein S und das den menschlichen Komplement-Cofaktor C4b-bindende Protein (C4bp) zu binden, aber spezifisch an den Komplex zu binden, ist und der andere ein Antikörper mit der Eigenschaft, an das den menschlichen Komplement-Cofaktor C4b-bindende Protein (C4bp) zu binden, ist.

5. Verfahren nach Anspruch 4, **dadurch gekennzeichnet,** daß der markierte zweite Antikörper mit einem Enzym, einer lumineszierenden Substanz oder einem Radioisotop markiert ist.

6. Verfahren nach Anspruch 4, **dadurch gekennzeichnet,** daß der markierte zweite Antikörper und die Testprobe gleichzeitig mit dem ersten Antikörper, der an einen unlöslichen, festen Träger fixiert ist, in Kontakt gebracht werden.

7. Reagenssystem zur immunologischen Bestimmung von freiem menschlichen Protein S in einer Testprobe, umfassend einen ersten Antikörper, der an einen unlöslichen, festen Träger fixiert ist, und einen markierten zweiten Antikörper, wobei der erste und der zweite Antikörper die Eigenschaft, an verschiedene Epitope von freiem menschlichen Protein S zu binden, besitzen und entweder der erste oder der zweite Antikörper ein monoclonaler Antikörper mit der Eigenschaft, nicht an einen Komplex aus dem menschlichen Protein S und dem den menschlichen Komplement-Cofaktor C4b-bindenden Protein (C4bp) zu binden, aber spezifisch an das freie menschliche Protein S zu binden, ist.

8. Reagenssystem zur immunologischen Bestimmung eines Komplexes aus menschlichem Protein S und einem den menschlichen Komplement-Cofaktor C4b-bindenden Protein (C4bp) in einer Testprobe, umfassend einen ersten Antikörper, der an einen unlöslichen, festen Träger gebunden ist und einen markierten zweiten Antikörper, wobei entweder der erste oder der zweite Antikörper ein monoclonaler Antikörper mit der Eigenschaft, nicht an freies menschliches Protein S und das den menschlichen Komplement-Cofaktor C4b-bindende Protein (C4bp) zu binden, aber spezifisch an den Komplex zu binden, ist und der andere ein Antikörper mit der Eigenschaft, an das den menschlichen Komplement-Cofaktor C4b-bindende Protein (C4bp) zu binden, ist.

9. Reagenssystem nach Anspruch 7 oder 8, **dadurch gekennzeichnet,** daß der unlösliche, feste Träger ein Plastikbehältnis, Plastikkugeln, Glaskugeln oder Metallteilchen ist.

10. Reagenssystem nach Anspruch 7 oder 8, **dadurch gekennzeichnet,** daß der markierte zweite Antikörper der monoclonale Antikörper, der mit einem Enzym, einer lumineszierenden Substanz oder einem Radioisotop markiert ist, ist.

**Revendications**

1. Méthode de dosage immunologique de la protéine S humaine libre dans un échantillon à analyser, qui comprend la mise en contact d'un anticorps primaire fixé à un support solide insoluble et d'un anticorps secondaire marqué avec l'échantillon à analyser, les anticorps primaire et secondaire ayant la propriété de se lier à des épitopes différents de la protéine S humaine libre, et l'un des anticorps primaire et secondaire étant un anticorps monoclonal ayant la propriété de ne pas se lier à un complexe de la protéine S humaine et de la protéine humaine liant le cofacteur C4b du complément (C4bp), mais de se lier spécifiquement à la protéine S humaine libre.

2. Méthode de la revendication 1, dans laquelle l'anticorps secondaire marqué est marqué par une enzyme, une substance luminescente ou un radio-isotope.

14

**3.** Méthode de la revendication 1, dans laquelle l'anticorps secondaire marqué et l'échantillon à analyser sont mis en contact simultanément avec l'anticorps primaire fixé à un support solide insoluble.

**4.** Méthode de dosage immunologique d'un complexe de protéine S humaine et de protéine humaine liant le cofacteur C4b du complément (C4bp) dans un échantillon à analyser, qui comprend la mise en contact d'un anticorps primaire fixé à un support solide insoluble et d'un anticorps secondaire marqué avec l'échantillon à analyser, l'un des anticorps primaire et secondaire étant un anticorps monoclonal ayant la propriété de ne pas se lier à la protéine S humaine libre et à la protéine humaine liant le cofacteur C4b du complément (C4bp) libre, mais de se lier spécifiquement audit complexe, et l'autre étant un anticorps ayant la propriété de se lier à la protéine humaine liant le cofacteur C4b du complément (C4bp).

**5.** Méthode de la revendication 4, dans laquelle l'anticorps secondaire marqué est marqué par une enzyme, une substance luminescente ou un radio-isotope.

**6.** Méthode de la revendication 4, dans laquelle l'anticorps secondaire marqué et l'échantillon à analyser sont mis en contact simultanément avec l'anticorps primaire fixé à un support solide insoluble.

**7.** Système de réactifs pour le dosage immunologique de la protéine S humaine libre dans un échantillon à analyser, comprenant un anticorps primaire fixé à un support solide insoluble et un anticorps secondaire marqué, dans lequel les anticorps primaire et secondaire ont la propriété de se lier à des épitopes différents de la protéine S humaine libre, et l'un des anticorps primaire et secondaire est un anticorps monoclonal ayant la propriété de ne pas se lier à un complexe de la protéine S humaine et de la protéine humaine liant le cofacteur C4b du complément (C4bp), mais de se lier spécifiquement à la protéine S humaine libre.

**8.** Système de réactifs pour le dosage immunologique d'un complexe de protéine S humaine et de protéine humaine liant le cofacteur C4b du complément (C4bp) dans un échantillon à analyser, comprenant un anticorps primaire fixé à un support solide insoluble et un anticorps secondaire marqué, dans lequel l'un des anticorps primaire et secondaire est un anticorps monoclonal ayant la propriété de ne pas se lier à la protéine S humaine libre et à la protéine humaine liant le cofacteur C4b du complément (C4bp) libre, mais de se lier spécifiquement audit complexe, et l'autre est un anticorps ayant la propriété de se lier à la protéine humaine liant le cofacteur C4b du complément (C4bp).

**9.** Système de réactifs de la revendication 7 ou 8, dans lequel le support solide insoluble est formé par un réceptacle de matière plastique, des perles de matière plastique, des perles de verre ou des particules métalliques.

**10.** Système de réactifs de la revendication 7 ou 8, dans lequel l'anticorps secondaire marqué est l'anticorps monoclonal marqué par une enzyme, une substance luminescente ou un radio-isotope.

Fig. 1

## *Fig. 2*

## *Fig. 3*

Fig. 4

Fig. 5

CHANGE IN ABSORBANCE ( $\Delta A_{415}$/min x $10^3$ )

CONCENTRATION OF PROTEIN S
(ng/ml)

CHANGE IN ABSORBANCE ( $\Delta A_{405}$/min x $10^3$ )

DILUTION RATIO OF PLASMA TAKEN
FROM A NORMAL HEALTHY PERSON

EP 0 271 810 B1

Fig. 6